(19) Europäisches Patentamt
European Patent Office
Office européen des brevets

(11) **EP 1 663 058 B1**

(12) **EUROPEAN PATENT SPECIFICATION**

(45) Date of publication and mention
of the grant of the patent:
**14.03.2012 Bulletin 2012/11**

(51) Int Cl.:
*A61C 17/22* (2006.01)   *A61C 17/34* (2006.01)
*A61N 5/06* (2006.01)   *H01L 25/075* (2006.01)

(21) Application number: **04788640.3**

(22) Date of filing: **09.09.2004**

(86) International application number:
**PCT/US2004/029334**

(87) International publication number:
**WO 2005/023131 (17.03.2005 Gazette 2005/11)**

(54) **ILLUMINATED ELECTRIC TOOTHBRUSHES**

BELEUCHTETE ELEKTRISCHE ZAHNBÜRSTEN

BROSSE A DENTS ELECTRIQUE LUMINESCENTE

(84) Designated Contracting States:
**AT BE BG CH CY CZ DE DK EE ES FI FR GB GR HU IE IT LI LU MC NL PL PT RO SE SI SK TR**

(30) Priority: **09.09.2003 US 501266 P
26.04.2004 US 832168
10.05.2004 US 842302
17.05.2004 US 847429
09.07.2004 US 847667
09.07.2004 US 887644
09.07.2004 US 888206**

(43) Date of publication of application:
**07.06.2006 Bulletin 2006/23**

(73) Proprietor: **THE PROCTER & GAMBLE COMPANY
Cincinnati, OH 45202 (US)**

(72) Inventors:
• **PINYAYEV, Aleksey, Mikhailovich
West Chester, OH 45069 (US)**
• **GHOSH, Chanchal, Kumar
West Chester, OH 45069 (US)**
• **CHAN, John, Geoffrey
Loveland, OH 45140 (US)**
• **PING, Wang,
Room 608, 7th Building
Beijing 100029 (CN)**

(74) Representative: **Zetterer, Gerd et al
Braun GmbH
Patentabteilung
Frankfurter Strasse 145
61476 Kronberg (DE)**

(56) References cited:
WO-A-95/10243       DE-U1- 29 908 517
US-A1- 2003 059 738   US-A1- 2003 104 340

• **PATENT ABSTRACTS OF JAPAN vol. 2002, no. 02, 2 April 2002 (2002-04-02) -& JP 2001 299454 A (MATSUSHITA ELECTRIC WORKS LTD), 30 October 2001 (2001-10-30)**

**Description**

**Field of the Invention**

The present invention relates to illuminated electric toothbrushes that utilize a light emitting diode, particularly a light emitting diode that illuminates the brushing area. More particularly, the present invention relates to the delivery of light of a particular luminous intensity or flux density that is in excess of the luminous intensity or flux density delivered by standard use of light emitting diodes.

**Background of the Invention**

Lighted toothbrushes have traditionally been manual brushes having a light disposed on or in the handle of the toothbrush with fiber optics carrying the light from the handle to the head of the toothbrush. However, light that is transmitted by fiber optics often diminishes in luminous intensity and/or flux density as it is transmitted. Therefore, it was desired to have a light disposed in or on the head of the toothbrush such that no fiber optic materials are necessary to transmit the light. Additionally, it was desired to have an electric lighted toothbrush.

In order for a light to be disposed on the head of a toothbrush, especially an electric toothbrush, the size must be minimized to allow sufficient space for bristles, and sufficient space for the mechanics of the electric toothbrush. A standard light emitting diode may be of the proper size; however such a device may not be able to deliver light having sufficient luminous intensity and/or flux density to provide an oral care benefit. A high power non-standard light emitting diode may be able to deliver the desired luminous intensity and/or flux density. However, the high power diodes use a.high current level, and thus generate a high level of heat Generating a high level of heat in the oral cavity can overheat the pulp chamber, which can result in pulpitis or other damage to oral tissues. Accordingly, there is a need for an illuminated electric toothbrush comprising a light emitting diode that emits light having a luminous intensity of at least about 7 candelas and/or flux density of at least about 30 mW/cm$^2$ which can safely be used safely in the oral cavity without damaging the teeth and/or other oral surfaces.

An electric toothbrush is known from document WO 95/10243 suggesting an array of a plurality of LEDs disposed on the brush head for emitting radiation at a wavelength suitable for treatment in the oral cavity. More precisely, the LEDs are preferably located outside the bristle area such that the LEDs are angled away from the bristle plain.

An electric toothbrush according to the preamble portion of claim 1 is known from document US20030059738 which discloses a rotating brush head and a light source, for example a diode which emits light from an outlet for activating an oral hygiene composition.

**Summary of the Invention**

It is an objective of the present invention to provide an improved illuminated electric toothbrush which, on the one hand, allows an effective whitening of the teeth and, on the other hand, can be used safely in the oral cavity without damaging the teeth and/or other oral surfaces.

This objective is achieved by an illuminated electric toothbrush as defined in claim 1. Preferred embodiments are laid down in the dependent claims.

Accordingly, it is suggested to provide an illuminated electric toothbrush comprising a handle, a head, and a neck extending between the handle and the head. The handle has a hollow interior region having a motor disposed therein. The head comprises bristles and at least one light emitting diode. The light emitting diode is powered by an electrical current and has a flux density at a representative tooth surface of greater than about 30 mW/cm$^2$ at a detector distance of about 0.68 cm and at a detector aperture area of about 0.46 cm$^2$. Further the light emitting diode has an emission temperature of less than about 43 °C at an emission distance of about 0.68 cm and an emission time of about 2 minutes. The illuminated electric toothbrush has one or more movable bristle holders comprising bristles disposed on the head; and the motor is operatively connected to the movable bristle holders by a drive shaft.

In one embodiment the desired flux density at a representative tooth surface is achieved by delivering a continuous forward current of greater than about 35 milliamps to the light emitting diode.

In another embodiment the desired flux density at a representative tooth surface is achieved by disposing a light emitting diode comprising at least two dices, one lens, one positive lead and one negative lead on the head of the illuminated electric toothbrush.

In yet another embodiment the desired flux density at a representative tooth surface level is achieved by delivering a pulsed or non-continuous forward current to the light emitting diode.

## Brief Description of the Drawings

The invention may take physical form in certain parts and arrangements of parts, embodiments of which will be described in detail in this specification and illustrated in the accompanying drawings which form a part hereof, and wherein:

FIG. 1 is a cross-sectional view of a light emitting diode.

FIG. 2-2c are cross-sectional views of a light emitting diode having more than one light emitter, and a single optical output.

FIG. 3 is a perspective view of an illuminated electric toothbrush in accordance with the present invention.

FIG. 4 is a top planar view of the electric toothbrush of FIG. 3.

FIG. 5 is a cross-sectional side elevational view of the electric toothbrush of FIG. 3.

FIG. 6 is a cross-sectional side view of the head of the electric toothbrush.

FIG. 6a is a cross-sectional side view of the head of the electric toothbrush.

FIG. 7 is a partial front elevational view of a head and neck of another embodiment of the present invention.

FIG. 8 is a partial front elevational view of a head and neck of yet another embodiment of the present invention.

FIG. 9 is a partial front elevational view of a head and neck of still another embodiment of the present invention.

FIG. 10 is a partial front elevational view of a head and neck of yet another embodiment of the present invention.

FIG. 11 is a partial front elevational view of a head and neck of yet another embodiment of the present invention.

FIG. 12 is a partial front elevational view of a head and neck of still another embodiment of the present invention.

FIG. 13 is a perspective view of another embodiment of the illuminated electric toothbrush of the present invention in which the toothbrush includes a head and neck that can be separated from the handle.

FIG. 14 and 15 are partial side elevational views illustrating installation of a replaceable head and neck onto a handle or body portion of the illuminated electric toothbrush of FIG. 11.

FIG. 16 is a schematic of an electrical configuration suitable for use with the present invention.

FIG. 17 is a graph of the spectral distribution for a variety of colors for light-emitting diodes that are suitable for use with the present invention.

FIG. 18 is a graph of the spectral distribution for a light-emitting diode that emits a white light that is suitable for use with the present invention.

FIG. 19 is a graph illustrating a light radiation pattern suitable for use with the present invention.

FIG. 20 is a diagram illustrating the geometry of the void between the light emitting diode and the surface to be exposed to light.

FIG. 21 is a diagram illustrating the test method for measuring average intensity of the light within a particular solid angle.

FIG. 22 is a diagram illustrating the test method for measuring the affect of the illuminating electric toothbrush on the temperature at the surface of the teeth.

## Detailed Description of the Embodiments

Generally, the present invention relates to an electric toothbrush having one or more light-emitting diodes ("LED") disposed on or in the head of the electric toothbrush. More specifically, the electric toothbrushes are used in personal hygiene to clean one's teeth and gums using a motorized movement, while the LEDs illuminate the region of brushing, including the teeth and/or gums. Additionally, the LEDs can provide an oral care benefit, such as whitening.

As used herein, the term "light" is intended to encompass the spectrum of both visible and non-visible (e.g., ultraviolet and infra-red) light. There are two systems for measuring light: radiometry and photometry, wherein radiometry is measurement of electromagnetic radiation within the frequency range between $3x10^{11}$ and $3X10^{16}$ Hz and photometry is the measurement of electromagnetic radiation that is detectable by the human eye. As known in the art, radiometric units include: Energy (Newton meter or joules), Power or Radiant Flux which is the flow of Energy with respect to time (joules/second or watts), Irradiance or Flux Density which is power per unit area (watts/$m^2$), Radiant Intensity which is power per unit solid angle (watts/steradian), and Radiance which is the power per unit projected area per unit solid angle (watts/$m^2$-steradian). Equivalent photometric units include: Power or Luminous Flux (lumen) and Luminous Intensity (lumen/sr or candela). Another characteristics of the light that will be discussed is the viewing or half angle. As described herein the half angle is two times the included angle (in degrees) between the peak and the point on one side of the beam axis at which the luminous intensity is fifty percent of the maximum or half of the beam angle. Yet another characteristic that will be discussed hereafter relates to the amount of heat or Emission Temperature (Celsius) which is generated by an LED at a tooth surface. Additionally, the total electric power consumed by the LED ("power dissipation") disposed on the head of the illuminated electric toothbrush will be characterized. For simplicity herein, units may be discussed in either radiometric units or photometric units, although radiometric units are preferred. Intensity can be either luminous intensity measured in candelas (or lumens/steradian), or flux density measured in Watts/meter$^2$.

All test methods described herein are performed when the illuminated electric toothbrush is operated at the current normally drawn to operate the device when the brush is fully charged and turned on, the bristles are moving, and the LED is illuminated.

Characteristics of the LEDs of the present invention are discussed more fully below.

A. Flux Density at a Representative Tooth Surface ("FDRT")

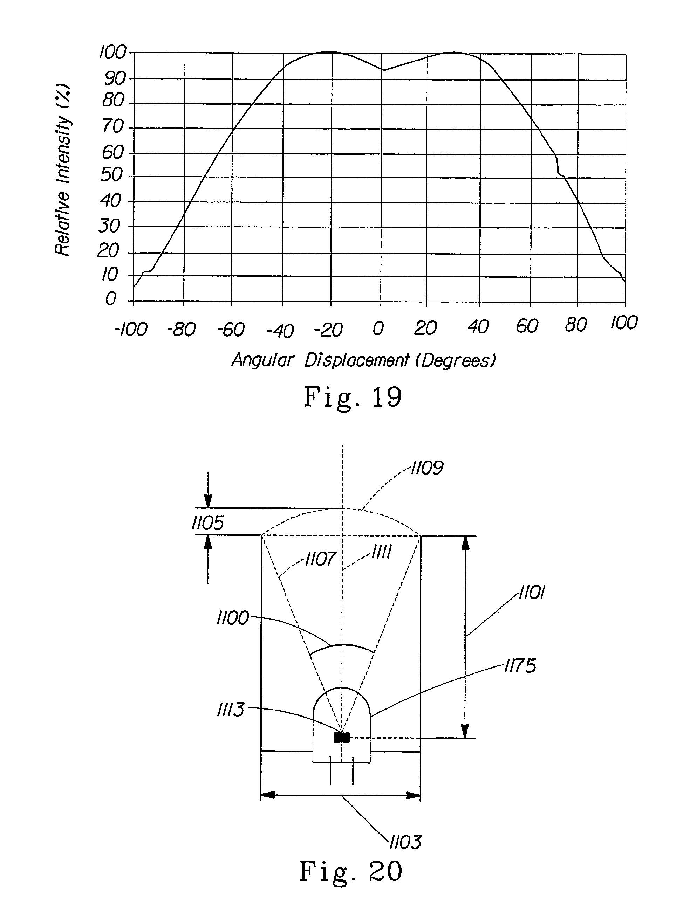

This test is intended to represent the radiant flux density projected onto a tooth surface in W/m$^2$. A detector calibrated in Watts having a detector aperture area of less than about 3.14, 1.77, 1.54, 1.33, 1.23, 1.13, 1.04, 0.95, 0.87, 0.79, 0.70, 0.64, 0.50, and/or 0.46 cm$^2$ and/or greater than about 0.28, 0.31, 0.32, 0.33, 0.38, 0.44, 0.46, and/or 0.50 cm$^2$ and a detector aperture diameter of at least about 0.60, 0.63, 0.64, 0.70, 0.76, 0.80, 0.90, 0.95, 1.00, 1.05, 1.10, 1.15, and/or 1 cm and/or less than about 2.0, 1.50, 1.40, 1.30, 1.25, 1.20, 1.15, 1.10, 1.00 cm, and the detector aperture has a distance ("detector distance") of greater than about 0.55, 0.60, 0.63, 0.64, 0.66, 0.68, 0.70, 0.72, 0.74, 0.76, 0.80, 0.85, 0.90 and/or 1.0 cm, and/or less than about 2.0, 1.5, 1.4, 1.3, 1.25, 1.20, 1.15, 1.10, 1.05 and/or 1.0 cm from the light emitting point of the LED. Traditionally, the detector comprises an iris that can provide a detector aperture area of the desired size. The LED should be positioned facing the detector aperture, and the mechanical axis of the LED should pass through the center of this detector aperture. The detector measures radiant flux (Watts) at the detector. The detector measures the radiant flux over the entire detector aperture area. Therefore, the resulting number is a total value of the radiant flux. The FDRT is the total value of the radiant flux divided by the Spherical Area of the cap **1109** (as shown in **FIG. 20** which illustrates the geometrical relationship between the LED and the surface to be exposed to light). The spherical area of the cap can be calculated by the following equations:

$$S = 2\pi R(R - l)$$

where:

$$R = \sqrt{l^2 + d^2/4}$$

S = spherical area of the cap
l = detector distance
d = diameter of detector aperture area.
FDRT = Total Radiant Flux (Watts) / S

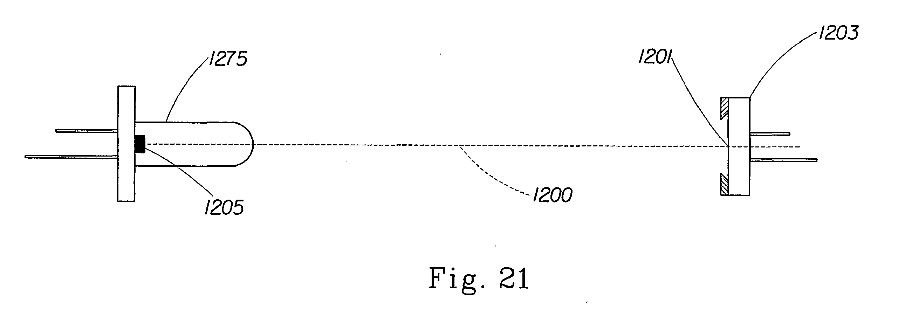

This radiant flux (Watts) is divided by the spherical area of the cap to result in flux density at a representative tooth surface (W/ m$^2$). An example of a device suitable for measuring the FDRT includes the OL 730CV Radiometer/Photometer manufactured by Optronic Laboratories, Inc. of Orlando, FL As illustrated in **FIG. 21** detector distance "l" (as shown at **1200**) is the distance between the light emitting point **1205** of LED **1275** and the entrance aperture **1201** of detector **1203.** This detector distance "1" (as shown at **1200**) is measured from the light emitting point **1205** of the LED **1275** to the plane of the detector aperture **1201** of the detector **1203.**

The FDRT of the inventive illuminated electric toothbrush is from at least about 30, 35, 40, 45, 50, 55, 60, 70, and/or 100 mW/cm$^2$ and/or less than about 300, 250, 200, 150, and/or 100 mW/cm$^2$ or any combination of these. It is believed that toothbrushes comprising LEDs that individually emit light at the aforementioned FDRT can result in whitening and other oral care benefits when used in the mouth alone or in combination with other oral care compositions. To achieve these oral care benefits at least one of the LEDs disposed on the head of the toothbrush must emit light having an FDRT of at least about 30 mW/cm$^2$. Light having a higher FDRT may also result in whitening or other oral care benefit, however if 300 mW/cm$^2$ is exceeded a user may need to take safety measures to prevent damage to the oral cavity.

B. Percent Total Luminous Flux within a Solid Angle

In one embodiment of the LED of the electric toothbrush, at least about 75%, 80%, 85%, 90%, 95%, 100% of the total power (watts) of the LED is contained within the solid angle with a vertex in the center of the LED of at least about 0, 0.5, 0.55, 0.6, 0.65, 0.7, 0.75, 0.8, 0.9, 0.95, and/or 1 steradian ("sr") and/or less than about 6.3, 5.5, 5, 4.5, 4, 3.5, 3, 2.5, 2, 1.5, 1.3, 1.2, 1.1, and/or 1 sr. The solid angle having a vertex in the light emitting point of the LED can be calculated using the equations below:

$$\alpha = S/R^2 = 2\pi h/R,$$

where:

$$h = R - a$$

and

$$R = \sqrt{a^2 + b^2/4}$$

$\alpha$ = solid angle (sr)
S = spherical area of the cap
a = axial distance
b = diameter of the dimensional area

These calculations are similar to the calculations as used above to calculate the FDRT, and the axial distance and dimensional area have similar values to the detector distance and detector area, however no detector is present in the calculation of the solid angle.

A diagram of the void space within which the LED emits light towards the surface to be exposed to light is shown in **FIG. 20.** The elements of the equation are depicted in **FIG. 20** wherein "$\alpha$" is the solid angle (shown at **1110**) with a vertex (shown at **1111**) in the light emitting point **1113** of the LED **1175.** "a" (illustrated in **FIG. 20** at **1101**) is the vertical distance between the emitting surface of the LED and the surface to be exposed to the light emitting from the LED ("axial distance"), "b" (shown at **1103**) is the diameter of a circular area comprising the LED, and "S" (shown at **1109**) is the spherical area of the cap. "h" (shown at **1105**) equals "R" (shown at **1107**) minus "a" (shown at **1101**). "b" can be at least about 0.60, 0.63, 0.64, 0.65, 0.70, 0.76, 0.80, 0.90, 0.95 and/or 1.00 cm, and/or less than about 2.0, 1.50, 1.40, 1.30, 1.25, 1.20, 1.15, 1.10, 1.05 and/or 1.00 cm. "a" can be greater than about 0.55, 0.60, 0.63, 0.64, 0.66, 0.68, 0.70, 0.72, 0.74, 0.76, 0.80, 0.85, 0.90 and/or 1.00 cm, and/or less than about 2.0, 1.50, 1.40, 1.30, 1.25, 1.20, 1.15, 1.10, 1.05 and/or 1.00 cm.

To determine the percent of power within the solid angle, first, the total power emitted from the LED must be measured, and second, the power within a particular solid angle area must be measured. Finally, the percent power within a particular solid angle is calculated. The total power emitted from the LED can be determined by either the goniophotometer method and/or the integrating sphere method. The goniophotometer method allows for the total radiant flux to be measured in Watts (when the goniophotometer is calibrated in Watts). The rotating detector of the goniophotometer scans the surface of a spherical shaped area surrounding the LED. The partial fluxes d$\Phi$ incident on each element dA of the surface represent a total radiant flux:

$$E(\theta,\varphi) = \frac{d\Phi}{dA}$$

Which can be weighted and integrated to give the value of the total radiant flux $\Phi$,

$$\Phi = \int_{(A)} E\,dA$$

Another method of measuring the total radiant flux from an LED is to use an integrating sphere (calibrated in Watts) to compare the tested LED to a standard LED with a similar spatial and spectral power distribution. If no perfectly matches standard is available, a correction for color can be calculated; however a correction for spatial power differences is more difficult to calculate. Most integrating spheres are no more than 10 cm in diameter. Therefore, an auxiliary LED of the same type should be inserted into the integrating sphere to allow for a correction to be applied for the self-absorption of the test LED. Spheres with two entrance and one exit port for the detector should work. Both of these methods are described in CIE 127 (1997) entitled "Measurement of LEDs", which is published by the International Commission of Illumination.

Second, the power within a particular solid angle is measured. To choose the solid angle within which the power is measured, the axial distance and diameter of dimensional area for the desired solid angle must be determined using the aforementioned equations. The axial distance value corresponds to the detector distance value, and the diameter of the dimensional area value corresponds to the detector aperture area value. By choosing these values when performing the test, the power within the desired solid angle is measured. If the detector has been calibrated in Watts, this results in total radiant flux within the desired solid angle.

The measurement of total radiant flux (within a particular solid angle) of the LED involves a detector calibrated in Watts having a circular aperture **1201** with an area of less than about 3.14, 1.77, 1.54, 1.33, 1.23, 1.13, 1.04, 0.95, 0.87, 0.79, 0.70, 0.64, 0.50, and/or 0.46 cm$^2$ and/or greater than about 0.28, 0.31, 0.32, 0.33, 0.38, 0.44, 0.46, and/or 0.50 cm$^2$, and a detector aperture diameter of at least about 0.60, 0.63, 0.64, 0.70, 0.76, 0.80, 0.90, 0.95, 1.00, 1.05, 1.10, 1.15, and/or 1 cm and/or less than about 2.0, 1.50, 1.40, 1.30, 1.25, 1.20, 1.15, 1.10, 1.00 cm. The LED should be positioned facing the detector aperture **1201** at a detector distance **1200** from the light emitting point **1205** of the LED **1275** of about 0.55, 0.60, 0.63, 0.64, 0.66, 0.68, 0.70, 0.72, 0.74, 0.76, 0.80, 0.85, 0.90 and/or 1.00 cm, and/or less than about 2.0, 1.50, 1.40, 1.30, 1.25, 1.20, 1.15, 1.10, 1.05 and/or 1.00 cm. The mechanical axis of the LED should pass through the center of this detector aperture.

Finally, the percentage of light emitted within the desired solid angle is calculated by the equation:

$$\left. \text{Total Radiant Flux Within the Desired Solid Angle} \middle/ \text{Total Radiant Flux} \right.$$

$$= \% \text{ of Light Emitted Within the Desired Solid Angle}$$

C. Half Angle and/or Viewing Angle

Another method for determining if a illuminated electric toothbrush emits light having the desired characteristics is to examine the half angle and/or viewing angle of the LED. As described herein the half angle is two times the included angle (in degrees) between the peak and the point on one side of the beam axis at which the luminous intensity is fifty percent of the maximum or half of the beam angle. This can also be referred to as the viewing angle. The smaller the half angle the more focused the light. The more focused the light emitting from the LED, the less light is needed to achieve the desired luminous intensity and/or FDRT. Having a more focused angle of light results in less light wasted from shining in non-preferred directions, i.e. shining into the bristles areas. If light is shined in non-preferred directions, more light will be required to achieve the desired luminous intensity or FDRT, often resulting in increased heat levels. Increased heat emission from the illuminated electric toothbrush can result in damage to the teeth and tissues in the oral cavity. The half angle $\left( 2\theta\dfrac{1}{2} \right)$ of the LED can be less than about 50°, 49°, 48°, 47°, 46°, 45°, 44°, 43°, 42°, 41°, 40°, 38°, 36°, 34°, 32°, 30°, and/or 28° and/or greater than about 0° and/or 5°.

D. Emission Temperature

Using an LED on the head of a toothbrush, which is then placed into the oral cavity for brushing and/or treating the teeth, may introduce heat as well as light into the oral cavity. The light can be absorbed by the surface of the tooth, thereby generating additional heat at the tooth surface. If heat is generated within the oral cavity, the pulp chamber of the tooth can be increased, which may result in pulpitis or other damage to the oral cavity. To avoid causing damage in the oral cavity, the temperature of the surface of the teeth should remain less than about 43°C, 40°C, 39°C, 38°C, 37°C, 36°C, 34°C, 30°C, and /or 25°C. If the temperature of the surface of the teeth is increased beyond the aforementioned temperatures, the pulp chamber of the tooth may be overheated, thereby resulting in pulpitis. Therefore, the light emitted by the illuminated electric toothbrush should not produce heat that raises the temperature of the surface of the teeth greater than about 43°C, 40°C, 39°C, 38°C, 37°C, 36°C, 34°C, 30°C, and /or 25°C. In one embodiment the temperature of the surface of the teeth is kept below about 43°C by using a standard LED and providing a continuous forward current less than about 200 milliamps ("mA") to the standard LED.

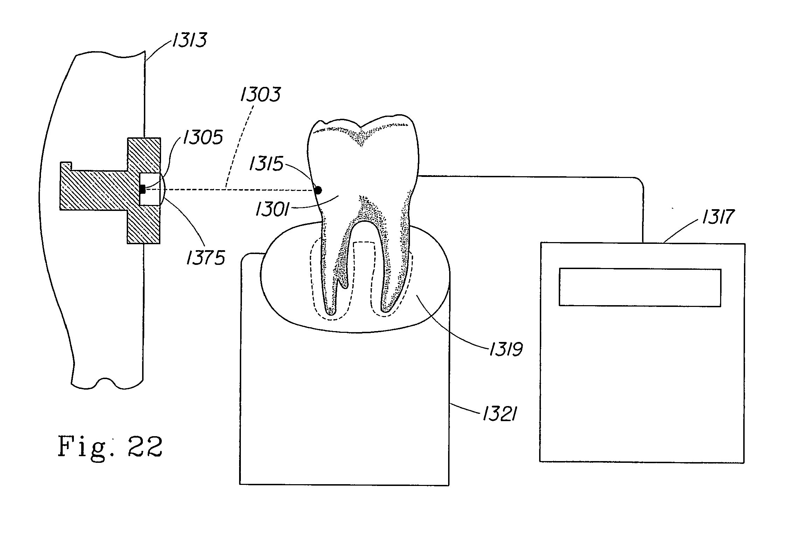

The temperature generated at the surface of the teeth resulting from exposure to light emitted from the illuminated electric toothbrush is the "emission temperature." The emission temperature can be measured by devices known in the art such as a thermo-couple **1315** (as shown in **FIG. 22**). One thermo-couple suitable for use in the present test method is the SC-GG-T-30-36 thermo-couple manufactured by Omega Engineering, Inc. The thermo-couple can be attached, preferably with adhesive, to the surface of the tooth exposed to light emitting from the LED. One suitable dental adhesive to use in this test method is Lucitone 199 manufactured by Dentsply. Alternatively, the temperature at the surface of the tooth can be measured after exposure to the light, so long as the thermo-couple is touched to the tooth and the temperature reading is completed within a testing time of less than about 10, 9, 8, 7, 6, 5, 4, 3, 2, 1 seconds of terminating exposure of the tooth to the light. One method of measuring temperature after exposure to the light is terminated is by using a standard cotton swab to apply and hold the thermo-couple on the tooth for the duration of the testing time to gather the temperature data. Additionally, a unit **1317** which translates the data from the thermo-couple into temperature in degrees can be used; hand held unit HH5-08 manufactured by Omega Engineering, Inc. is suitable to be used with aforementioned thermo-couple to translate data received from the thermo-couple into temperature in degrees. This testing is performed in vitro on standard extracted human or bovine tooth **1301** samples, within an incubator set at 32°C. The test is performed within a incubator set at 32°C to replicate the normal base temperature of a tooth placed in the mouth. A suitable incubator for this test is the THELCO 3DG, catalog #51221122 available from the Jouan Group of Companies. The tooth is placed in cast aluminum stand **1319** comprising a piece of cast aluminum with a space removed for placement of the tooth. The cast aluminum stand **1319** connects the tooth **1301** to a heat sink **1321.** A heat sink suitable for use in the present test method includes heat sink 11-5602-48 VIS #031608 manufactured by Aavid Thermalloy. A power supply (not shown) can be provided to the heat sink. The "emission distance" is the distance **1303** between the light emitting point **1305** of the LED **1375** and the surface of the tooth **1301**. The emission distance **1303** can be less than about 3.14, 1.77, 1.54, 1.33, 1.23, 1.13, 1.04, 0.95, 0.87, 0.79, 0.70, 0.64, 0.50, and/or 0.46 cm and/or greater than about 0.28, 0.31, 0.32, 0.33, 0.38, 0.44, 0.46, and/or 0.50 cm from the surface of the tooth. The light emitting point **1305** of the LED **1375** is placed at an emission distance of less than about 3.14, 1.77, 1.54, 1.33, 1.23, 1.13, 1.04, 0.95, 0.87, 0.79, 0.70, 0.64, 0.50, and/or 0.46 cm and/or greater than about 0.28, 0.31, 0.32, 0.33, 0.38, 0.44, 0.46, and/or 0.50 cm from the surface of the tooth **1301,** and the illuminated electric toothbrush **1313** is turned on; thereby operating the LED **1375** and illuminating the surface of the tooth **1301.** The tooth **1301** is then exposed to light emitting from the LED **1375** for an emission time of less than about 15, 14, 13, 12, 11, 10, 9, 8, 7, 6, 5, 4, 3, 2, 1, and/or 0 minutes and the temperature of the tooth **1301** is measured by the standard thermo-couple **1315**. The thermo-couple can be attached to a separate hand-held unit **1317** to translate the readings from the thermo-couple **1315** into temperature readings. The emission temperature should not exceed about 43°C, 40°C, 39°C, 38°C, 37°C, 36°C, 34°C, 30°C, and /or 25°C.

E. Power Dissipation

Additionally, to avoid damage to the oral cavity due to excessive heat generation, the total electric power consumed ("power dissipation") by the LED disposed on the head of the illuminated electric toothbrush should not exceed about 2, 1.5, 1, 0.95, 0.9, 0.85, 0.8, 0.75, 0.7, 0.5, 0.4, 0.3, 0.2, 0.1 Watts ("W").

F. Examples of Embodiments of the Invention

Luminous intensity of at least about 7 candelas and/or FDRT of at least about 30 mW/cm$^2$ can be achieved in the inventive illuminated electric toothbrush comprising a standard LED by increasing the forward current beyond that recommended by the manufacturer ("overpowering"), including more than one light emitter in the LED, and/or pulsing the light emitted from the LED, or any combination of these. Overpowering of the LED can shorten the life span of the LED. The amount the life span of the LED is shortened depends on the level of current used to overpower the LED and the characteristics of LED. However, this shortened life span will still exceed what is needed for use on a toothbrush, as a toothbrush is a disposable and/or replaceable item. In one embodiment the LED is disposed on a replaceable portion of the toothbrush, and can therefore be replaced if desired.

As used herein, the term "light" is intended to encompass the spectrum of both visible and non-visible (e.g., ultraviolet and infra-red) light. This spectrum may extend from light having a dominant or centroid wavelength of about 10 nm (far ultraviolet) to light having a centroid wavelength of 10$^6$ nm (infrared), or the spectrum may include visible light having a centroid wavelength between about 370 nm and about 770 nm. Further, the spectrum may include visible light having a centroid wavelength between about 370 to about 500. As used herein, the term "centroid wavelength" is intended to refer to the wavelength which represents the perceived color of the light. This may be different than the peak wavelength which is the wavelength at which the radiant intensity of the LED is maximum. For LEDs, the dominant or centroid wavelength can be determined by the equations:

$$\lambda_c = \int_{\lambda\min}^{\lambda\max} I(\lambda)\cdot\lambda\cdot dl \Big/ \int_{\lambda\min}^{\lambda\max} I(\lambda)\cdot d\lambda$$

For continuous spectrums, and

$$\lambda_c = \sum_i I_i \lambda_i \Big/ \sum_i I_i$$

For discrete spectrums.

Wherein $I$ is illumination intensity and $\lambda$ is wavelength.

These equations are further described in CIE 127 (1997) entitled "Measurement of LEDs", which is published by the International Commission of Illumination. The spectral (e.g., peak wavelength), photometric (e.g., luminous intensity), radiometric (e.g., radiant intensity), and colorimetric (e.g., dominant wavelength) characteristics of the LEDs can be measured using devices known in the art, such as OL 730CV Radiometer/Photometer manufactured by Optronic Laboratories, Inc. of Orlando, FL Some light may not have a dominant or centroid wavelength (e.g., white light).

The inventive illuminated electric toothbrush comprises LEDs that emit light having a luminous intensity of at least about 7, 10, 15, 20, 30, and/or 40 and/or less than about 60, 50, 45, and/or 40 Candelas or any combination of these, or a FDRT of at least about 30, 35, 40, 45, 50, 55, 60, 70, and/or 100 mW/cm$^2$ and/or less than about 300, 250, 200, 150, and/or 100 mW/cm$^2$ or any combination of these.

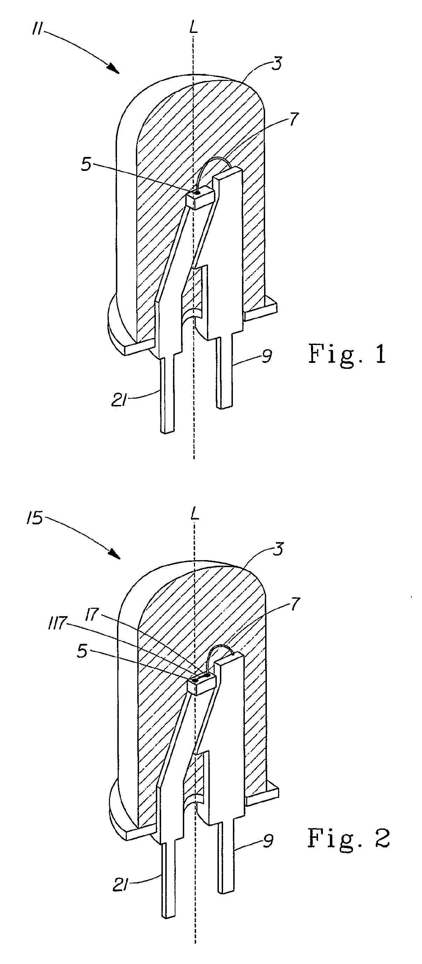

One embodiment of the illuminated electric toothbrush comprises an LED as shown in **FIG. 1. FIG. 1** shows a cross section of LED package **11** comprising a lens **3**, a single light emitting dice **5**, a wire bonding **7**, a positive lead **21** and negative lead **9**, and a Longitudinal axis **L.** Various types of semi-conductor substrates having light emitting properties can be used in LEDs of the claimed invention. One type of semi-conductor substrate having a light emitting property is a dice. A dice is a single semi-conductor substrate having light emitting properties. It is contemplated that the LED disposed on the head of the inventive illuminated electric toothbrush can comprise any type of semi-conductor substrate having light emitting properties, including but not limited to a dice, so long as the illuminated electric toothbrush provides light having the desired properties described herein. The LED can have a diameter of at least about 0.5, 1, 2, 3, 4, 5, and/or 6 mm and/or less than about 5, 10, 15, and/or 20 mm.

Light can emit from many surfaces of the light emitting point of an LED. However, for simplicity hereinafter all measurements of the distance from the light emitting point and/or surface of the LED refer to the front surface of the semi-conductor substrate, such as the front surface of the dice **5.** If the LED has multiple dices, and therefore multiple front surfaces of the semi-conductor substrate, the distance from the light emitting point of an LED should be the average of the distances from the front surface of the semi-conductor substrates. Light emits from a surface of the dice and is directed to the lens **3** of the LED. Therefore, to measure a distance from the light emitting point of a semi-conductor substrate, the front surface of the light emitting element of the semi-conductor substrate must be identified. In one embodiment of the illuminating electric toothbrush the front surface of the light emitting element of the LED is the surface of the dice **5** (as shown in **FIG. 1**). Therefore, all measurements of distance from this embodiment of a light emitting surface begin with the front surface of dice **5**.

Overpowering the LED results in the desired luminous intensity and/or FDRT because, luminous intensity and/or FDRT of a LED increases, within limits, as forward current input increases. Therefore, the luminous intensity and/or FDRT levels desired for the inventive illuminated electric toothbrush can be achieved by increasing the current to a standard LED beyond that recommended by the manufacturer. Increasing the current twice the maximum recommended by the manufacturer will almost double the luminous intensity and/or FDRT, while still resulting in a lifespan of the LED acceptable for use in an illuminated electric toothbrush. A standard driver can be used to deliver the chosen current level to achieve the desired luminous intensity and/or FDRT. A voltage or current driver suitable for use with the present invention is the ZXSC310 Single or Multi Cell LED Driver manufactured by Zetex Semiconductors, Oldham, UK. The minimum current to achieve the desired luminous intensity and/or FDRT can be greater than the maximum current recommended by the manufacturer for continuous operation, two times the maximum recommended by the manufacturer for continuous operation, or three times the maximum recommended by the manufacturer for pulsed operation. At a maximum the current can be increased to the level which causes immediate failure of the LED. One embodiment of the invention comprises a standard LED which delivers the desired luminous intensity and/or FDRT via a continuous forward current greater than about 35 mA, 40 mA, 45 mA, 50 mA, 55 mA, 60 mA, 65 mA, 70 mA, 75 mA, 80 mA, 90 mA, 100 mA, 150 mA and/or 200 mA and/or less than about 700 mA, 600 mA, 500 mA, 400 mA, 300 mA, 250 mA, 200 mA, 150 mA, 100 mA, 90 mA, 80 mA, 75 mA, 70 mA, 65 mA, 60 mA, 55 mA, 50 mA, 45 mA, 40 mA, and/or 35 mA. In one embodiment the minimum continuous current level can be the maximum continuous current rating for continuous operation, and the maximum continuous current level can be about the current causing immediate failure of the LED. Although the luminous intensity and/or FDRT does increase as the current increases, there is a point at which this correlation levels out, and further current increase does not result in luminous intensity and/or FDRT increase. This exact point depends on the properties and design of the LED. Additionally, as time passes and the LED is exposed to currents beyond that recommended by the manufacturer, the luminous intensity and/or FDRT begins to fade. One way of maintaining the desired luminous intensity and/or FDRT includes, but is not limited to, further increasing the current in order to maintain the same luminous intensity and/or FDRT. Although the current is increased to the standard LED to achieve the desired luminous intensity and/or FDRT, the current used is still lower than traditionally used for high power non-standard LEDs. Therefore, the heat generated by the standard LEDs does not increase the temperature of the surface of the teeth above about 43 °C.

Stabilizing the current of the LED in a standard driver design does partially stabilize the luminous intensity and/or FDRT over time since the current stays the same as the LED decays. However, as the LED decays the current may need to be increased to maintain the same level of luminous intensity and/or FDRT. One way of maintaining constant luminous intensity and/or FDRT as the LED decays is to measure the luminous intensity and/or FDRT emitted from the LED with a built in sensor and adjust the current according to the measured value. Adjusting the current as the LED decays results in an illuminated electric toothbrush which continues to deliver light at the specified luminous intensity and/or FDRT over time. Another way of maintaining approximately the same luminous intensity and/or FDRT without including a built in sensor, is to include a timing circuit which increases the current to the LED over time as the LED decays. This can maintain approximated steady luminous intensity and/or FDRT via a simple design, and with minimal additional expense. A voltage or current driver suitable for use with the present invention is the ZXSC310 Single or Multi Cell LED Driver manufactured by Zetex Semiconductors, Oldham, UK.

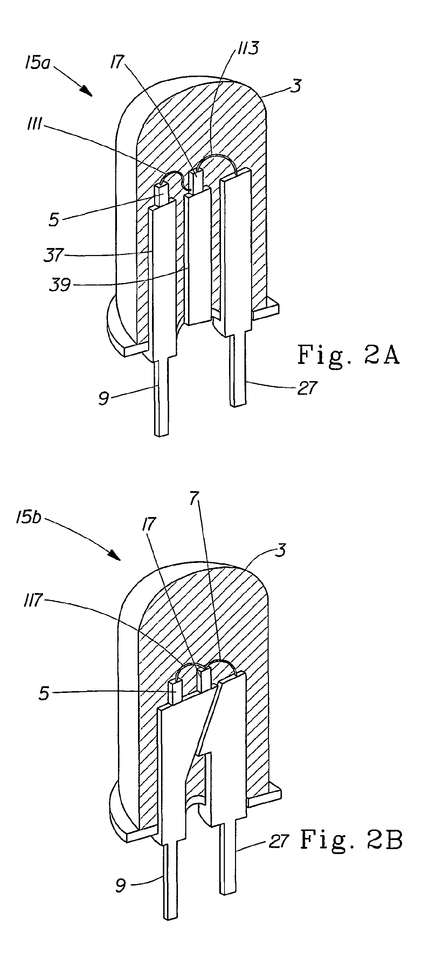

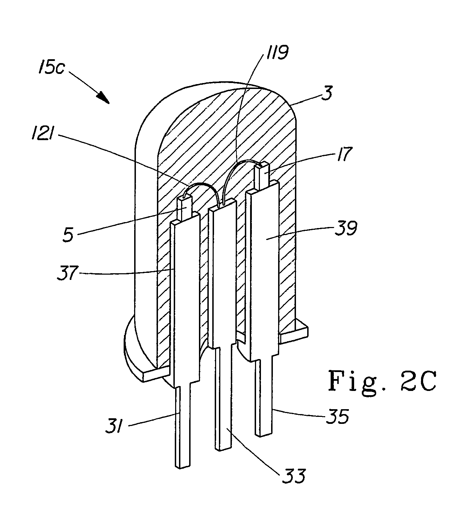

FIG. 2 shows another means for achieving the levels of luminous intensity and/or FDRT in the inventive illuminated electric toothbrush by including more than one light emitter such as multiple dices. The following embodiments illustrate LEDs having two semi-conductor substrates that emit light, such as dices, however it is contemplated that the LED could comprise more than two dices. This embodiment 15 of the invention has a single light output, the lens 3, and one positive lead 21 and one negative lead 9. However, this single standard LED package contains more than one light emitter and more than one semi-conductor substrate, and can have more than two leads. All light from the light emitting sources is combined to result in a single light output at lens 3 of LED package 15. The single LED package 15 has multiple light emitting dices 5 and 17 and a wire bonding 7 and 117. Embodiment 15 shows a connection between the dices 117. This connection can be either a parallel connection or a serial connection. FIG. 2a illustrates multiple dices connected in series. This embodiment 15a of the invention has a single light output, the lens 3, and one positive lead 9 and one negative lead 27. However, this single standard LED package contains more than one dice 5 and 17, with each dice having an individual pedestal 37 and 39. The dices have a serial connection, wire bonding 111 connects the top of dices 5 to the bottom of dices 17, and wire bonding 113 connects the top of dices 17 to the negative lead 27. All light from the light emitting sources is combined to result in a single light output at lens 3 of LED package 15a. FIG. 2b illustrates multiple dices connected in parallel. This embodiment 15b of the invention has a single light output, the lens 3, and one positive lead 9, and one negative lead 27. The dices have a parallel connection, wire bonding 117 connects the top of dices 5 to the top of dices 17, and wire bonding 7 connects the top of dices 17 to the top of the common negative lead 27. All light from the light emitting sources is combined to result in a single light output at lens 3 of LED package 15b. In another embodiment 15c (as shown in FIG. 2c) of this multi-dice LED, the LED comprises a lens 3, two semiconductor substrates, dices 5 and 17 shown connected in parallel, wire bondings 119 and 121, one positive lead 33, and two negative leads 31 and 35. This LED also emits light from a single light output, the lens 3. Each dice having an individual pedestal 37 and 39. It is also contemplated that the LED can comprise two positive leads, and one negative lead; and further this embodiment of the LED can be connected in series. Additionally, the LED can comprise more than two semi-conductor substrates having light emitting properties, and the LED can comprise more than two leads. The LED can have a common or shared lead, or can have individual leads for each semi-conductor substrate having light emitting properties. Further, each semi-conductor substrate having light emitting properties can be individually powered by a separate power source, such as a battery.

These dices can be electrically connected in parallel or in series. When they are connected in series, all current considerations are the same as for one single dice. The total voltage will be approximately n x $V_i$ where n = number of dices, and $V_i$ = forward voltage for a single dices. If the dices are connected in parallel, the total current will be approximately n x $I_i$ and the total voltage approximately that of a single dice. Serial connection works well because it adjusts for differences between the dices. When the dices are connected in series, they automatically adjust their forward voltages and their luminous intensity and/or FDRT become very close. In either arrangement the two dices LED has approximately the luminous intensity and/or FDRT of 1.6 x $P_i$, where $P_i$ is luminous intensity and/or FDRT of a single dice. A three dices LED will likely have the luminous intensity and/or FDRT of about 2.26 x $P_i$. (Interference between the dices can prevent the luminous intensity and/or FDRT calculation from being a multiplier by the number of dice.) These dices can deliver the same color of light, or they can have different colors of light. However, if each individual light emitter emits the same light, the luminous intensity and/or FDRT of that color light from that one single LED is greater than a single standard LED emitting light of one color. Each of the individual light emitters can emit light having a wavelength of from about 440 to about 480 nm. A single LED could also contain two dices emitting different colors of light, for example a wavelength selected from the range of greater than about 370, 380, 390, 400, 425, 440, 450, 475, 480 and/or less than about 500 nanometers. The dices could also be selected such that the dices emit light of a different wavelength within the same color range; for example the dices could emit light having different wavelengths that result in the color blue. Further, the combination of the different wavelengths of light at the single optical output of the LED (the lens) could result in a specific combination of colors that delivers an oral care benefit. For example, two different compositions can be applied to the teeth, each of which reacts to a different wavelength of light. Additionally, different wavelengths of light may result in different reactions within the oral cavity; one wavelength of light may kill bacteria, another wavelength of light may whiten the teeth. Some colors are difficult to achieve by a single wavelength of light; this invention can be used to produce light of one of these unique colors. Thus the combination of different colors at the single optical output may result in a color that cannot be achieved by one dice alone. Therefore, using different colors could result in one or more oral care benefits that a single wavelength of a single color could not achieve.

Yet another means for achieving the luminous intensity and/or FDRT of the inventive illuminated electric toothbrush includes providing a non-continuous or pulsing current to the LED which results in pulsed or non-continuous light. This embodiment of the invention comprises a standard LED which provides the desired luminous intensity and/or FDRT level via a pulse forward current greater than about 100 mA, 125 mA, 150 mA, 175 mA, 200 mA, 225 mA, 250 mA, 275 mA, 300 mA, 325 mA, 350 mA, and/or 375 mA and/or less than about 900 mA, 800 mA, 700 mA, 600 mA, 500 mA, 400 mA, 375 mA, 350 mA, 325 mA, 300 mA, 275 mA, 250 mA, 225 mA, 200 mA, 175 mA, 150 mA, 125 mA, and/or 100 mA. In one embodiment the pulsed forward current is greater than about the maximum current rating for pulsed operation and less than about the current causing immediate failure of the LED. The minimum luminous intensity and/or FDRT of the light pulses can be that of continuous light, and the maximum luminous intensity and/or FDRT is Pc/Q where Pc is the luminous intensity and/or FDRT of continuous light and Q is the cycle ratio. The cycle ratio equals the duration of the pulse divided by the time period between pulses. The inventive cycle ratio is from about 0.01, 0.10, 0.25, 0.40, and/or 0.50 to about 0.50, 0.60 0.75, 0.80, and/or 0.99. The frequency of the light pulses can be about 0.01 Hz, 1Hz, 10Hz, 100Hz, 500 Hz, or 1MHz to about 1MHz, 10 MHz, 100MHz, 500 MHz, 1 GHz, or 10GHz. The current amplitude for the pulsed operation of the LED can go from about $I_{maxp}$ to about 10 $I_{maxp}$, where $I_{maxp}$ is the absolute maximum current rating for pulsed operation, or from about $I_{max}$ to about 20 $I_{map}$, where $I_{max}$ is the maximum current rating for continuous operation. Pulsing the current to the LED results in a reduction of the LED's power dissipation, and therefore prolonged battery life, as well as an increase in light brightness, and/or luminous intensity and/or FDRT. The improved battery life and increased brightness can vary depending on the properties and design of the LED.

In one embodiment, the illuminated electric toothbrush includes an elongated body portion or handle, a head, and a neck extending between the head and the handle. One or more LEDs are provided on the head, preferably adjacent to, on, and/or in, one or more static or moving bristle holders having a plurality of bristles thereon. The bristles may be formed into one or groups of tufts.

The head includes a longitudinal axis, one or more moving bristle holders and, optionally, one or more static or fixed bristle holders. The moving bristle holders may rotate, swivel, gyrate, oscillate, linearly reciprocate, or undergo any combination of motions. The type of motion provided by the electric toothbrushes of the present invention can be widely varied. The static bristle holders and the arrangement of the static bristles disposed thereon can also be widely varied. For example, the static bristles might partially or wholly circumscribe the moving bristle holders or may be disposed in a gap between the moving bristle holders. Examples of some bristle holder motions and bristle arrangements suitable for use with the present invention are described in US 20030126699; US 20030084525; US 20030084524; US 20030084526; and WO 03/063723; and WO 03/063722. The bristles can be made from conventional non-elastomeric materials, such as polyethylene, or can be made from elastomeric materials such as natural or synthetic rubbers, polyolefms, polyetheramides, polyesters, styrenic polymers, polyurethanes, etc., or a combination of materials.

The handle has a hollow portion with a motor disposed therein that is operatively connected to the moving bristle holders. A motor is operatively connected to the moving bristle holder when some action by the motor results in a response in the moving bristle holder. A shaft may extend from the motor through the neck and into at least a portion of the head. The shaft may rotate, oscillate, linearly reciprocate, gyrate, vibrate or orbit when driven by the motor in order to impart one or more motions to the moving bristle holders. A gearing arrangement can be provided between the motor and the shaft or between the shaft and the moving bristle holders in order to impart motion thereto. Exemplary shaft and/or gearing arrangements are shown in USPNs 6,360,395 and 5,617,601 as well as in other patents and patent publications referenced herein. The handle also has a power source, such as one or more batteries, disposed therein for powering the motor and the LED. Alternatively, the electric toothbrush may be connected to an external power source for powering the motor. A switch is disposed on the handle for activating the motor and/or LEDs. The LEDs can be energized whenever the motor is activated. However, the toothbrush also can have more than one switch to activate the LEDs and/or the movable bristle holder.

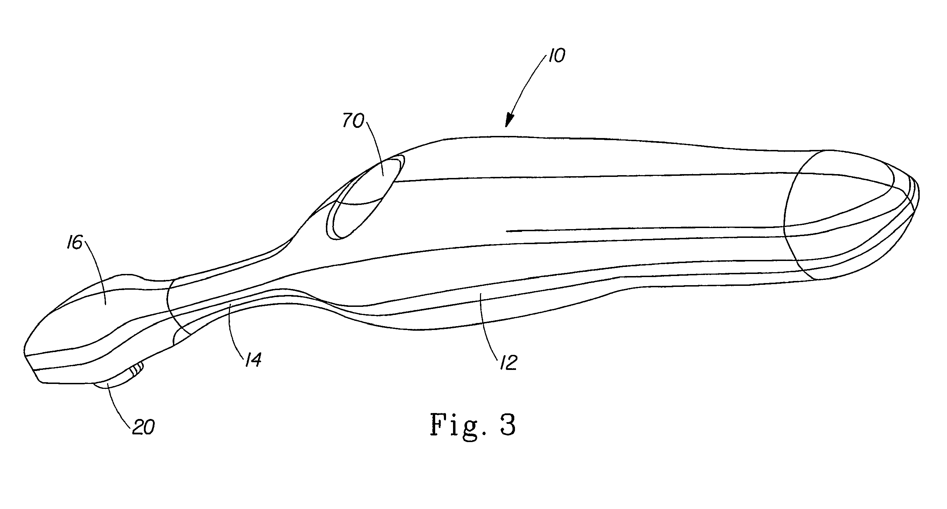

**FIG. 3** shows an illuminated electric toothbrush **10** according to the present invention. The electric toothbrush can be used for personal hygiene such as brushing one's teeth and gums. As shown in **FIG. 3,** the electric toothbrush includes a handle **12** a gripping portion **70,** and a neck **14** attached to the handle **12.** A head **16** is attached to neck **14.** Typically, the head is larger than the neck **14,** which is also typically smaller than the handle **12.**

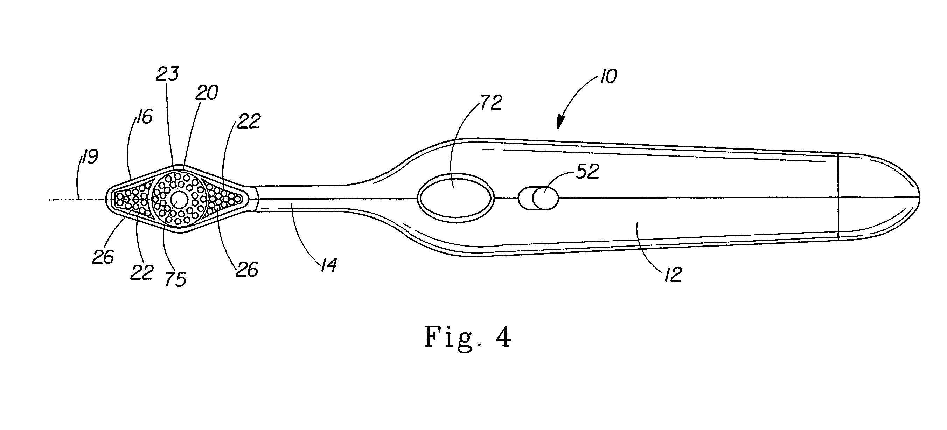

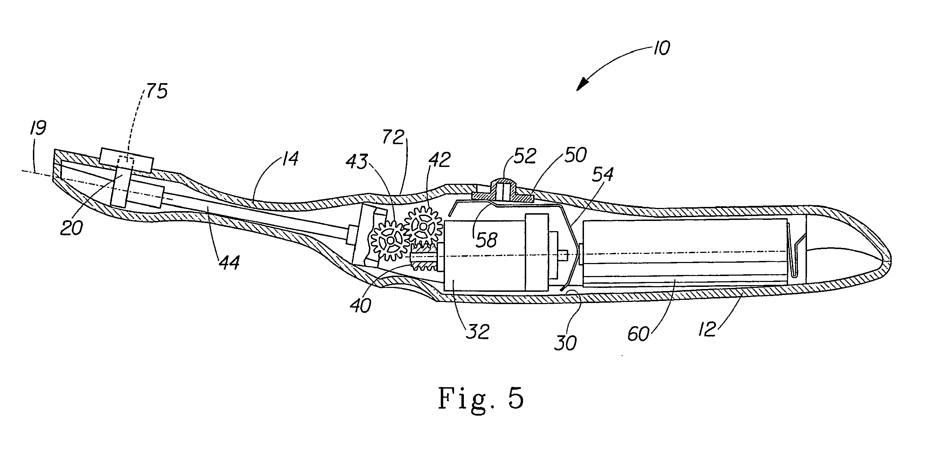

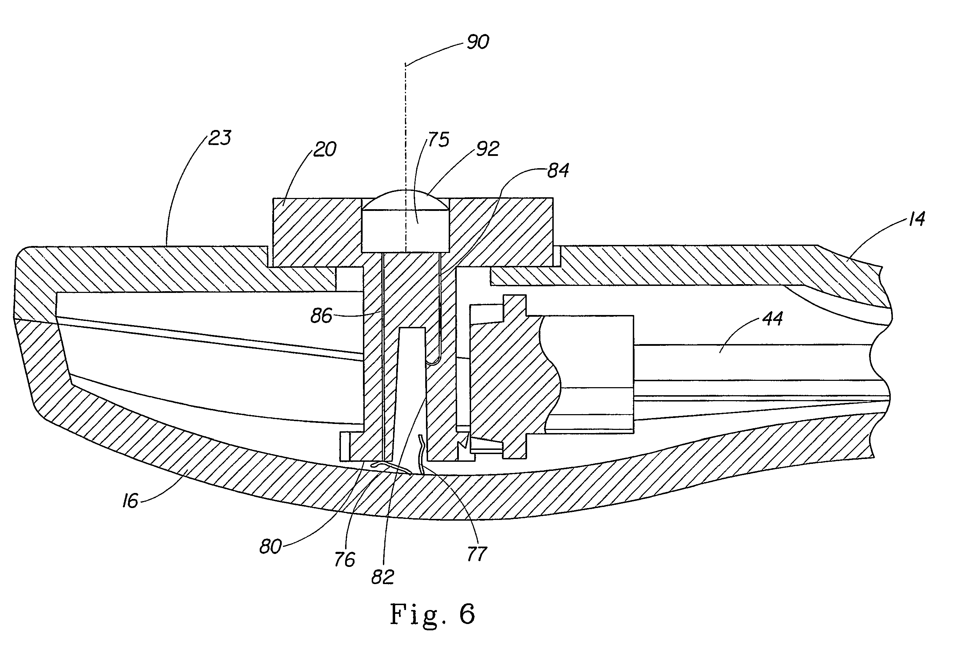

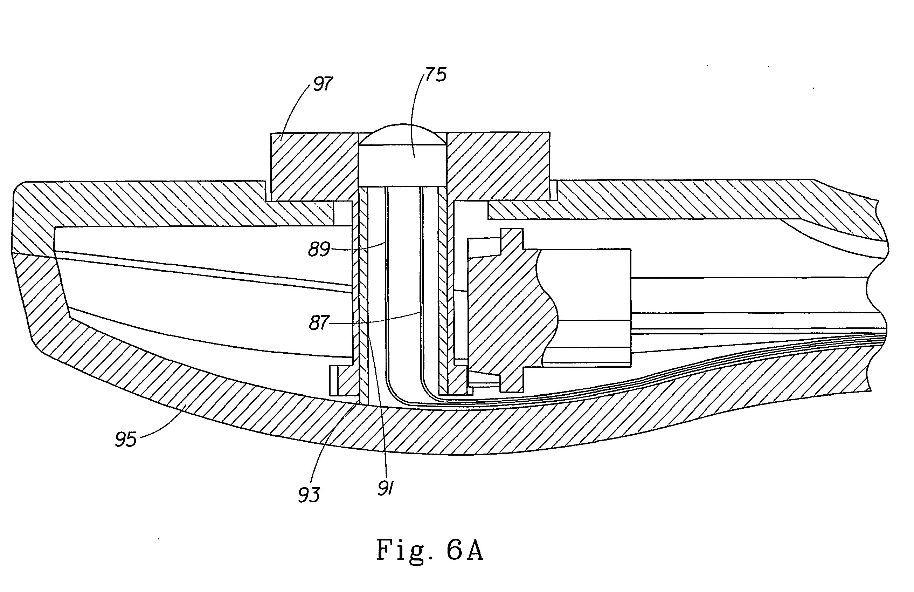

Referring now to **FIG. 4,** the toothbrush **10** comprises head **16,** longitudinal axis **19,** a handle **12,** a neck **14,** gripping region **72,** switch **52,** a moving bristle holder **20** and static bristle holders **22** having bristles **26** disposed thereon. The static bristle holders **22** are located on opposite sides of the moving bristle holder **20.** The moving bristle holder **20** is located at the center of the head **16.** The moving bristle holder preferably oscillates about an axis approximately normal to the longitudinal axis **19** of the head **16,** although other motions may be provided as previously described. As shown in **FIG. 5,** the handle **12** further includes a hollow portion **30** which houses a motor **32.** The motor **32** powers the moving bristle holder **20** through a rotatable shaft **44.** A gearing arrangement is operatively interconnected between the shaft **44** and the motor **32.** The gearing arrangement includes a worm gear **40** and a pair of step gears **42, 43.** The motor **32** is operatively connected to the worm gear **40.** Step gear **42** is operatively connected to step gear **43** and the worm gear **40.** A LED **75** is provided that is disposed in the interior of the moving bristle holder **20.** The LED **75** is mounted or secured to the moving bristle holder **20** so that LED **75** moves with moving bristle holder **20.** As shown in **FIG. 6,** electric power is provided to the LED **75** by the use of a pair of electrical contacts **76** and **77** that slidingly contact dedicated contact portions defined along the underside of the moving bristle holder **20.** Electrical wires (not shown) may be provided from the switch and power source to the contacts **76** and **77** for conducting electricity from the power source to the LED. The wires may run from the handle **12** through the neck **14** to the head **16.** Preferably, the wires are disposed adjacent the interior wall of the neck **14** so that they do not interfere with the movement of the shaft **44.** Alternatively, the wires may be embedded within the neck **14.**

It is contemplated that circular electrically conductive contact regions **80** and **82** could be provided along the exterior of the moving bristle holder **20,** which regions would be in electrical communication with the pair of fixed contacts **76** and **77** provided within the interior of the head. The electrically conductive contact regions **80** and **82** are insulated from each other by a non-conductive material. Electrical leads **84** and **86** can be provided from the electrically conductive contact regions to the LED. **FIG. 4** illustrates the LED **75** disposed on or within the moving bristle holder **20.** In this embodiment the LED is fixedly attached to the moving bristle holder **20** and therefore moves with the bristle holder. Preferably the tip of the LED is flush with the top surface **23** of the moving bristle holder **20,** although it may extend above the top surface **23** if desired. Additional LEDs can be provided in or on the static bristle holders **22. FIG. 6a** shows a stationary LED **75** that is connected to a pillar **91** that is stationary and fixed to the head **95** at **93** of the toothbrush. The moving bristle holder **97** oscillates or rotates around the stationary LED. The positive lead **87** and the negative lead **89** can run from the LED **75** through the pillar **91** and then down the length of the head **95** of the toothbrush to the power source (not shown).

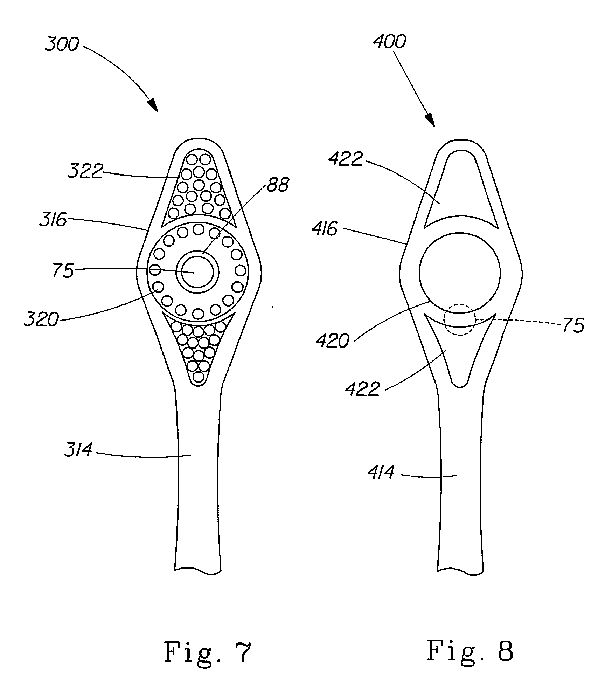

In another embodiment, the LED **75** is disposed within an aperture or hole **88** that extends through the moving bristle holder **320,** as best seen in embodiment **300** as shown in **FIG. 7,** so that the LED is stationary and the moving bristle holder **320** oscillates or rotates about the stationary LED **75.** In this embodiment, the LED **75** is fixedly secured to the head **316.** The LED **75** might extend partially through the hole **88** or it may be disposed below the lower surface of the moving bristle holder **320** so that it is completely contained within the head **316.** The centerline or axis of the LED **75** may also be the axis of rotation or oscillation for the moving bristle holder **320.** Neck **314** extends between head **316** and a handle (not shown). The head **316** further comprises static bristles **322.**

In each of the above-described embodiments, the LED is disposed in, on, below or directly adjacent the moving and/or static bristle holders so that the light is directed onto the brushing area as efficiently as possible. Further, the LEDs are preferably arranged so that the principle direction of light emission is generally perpendicular to the top surface of the bristle holders and/or generally parallel to the direction of the bristles of the bristle holder. In other words, the LED is preferably arranged so that the centerline **90** of the LED is generally perpendicular to the top surface of the head and/or bristle holder, as best seen in **FIG. 6.** The centerline **90** typically passes through the lens **92** or aperture of the LED. When the LED is **disposed** within, on, or below a moving and/or static bristle holder, a cylindrical region or volume about the centerline **90** of the LED can be substantially devoid of bristles. The area substantially devoid of bristles can be larger and/or smaller depending on the size of the head of the toothbrush, and/or the number of bristles removed in the area surrounding the LED. The area substantially devoid of bristles can be greater than about 0.55, 0.60, 0.63, 0.64, 0.66, 0.68, 0.70, 0.72, 0.74, 0.76, 0.80, 0.85, 0.90 and/or 1.0 cm, and/or less than about 2.0, 1.5, 1.4, 1.3, 1.25, 1.20, 1.15, 1.10, 1.05 and/or 1.0 cm. The moving bristle holder still, however, preferably has at least one ring of bristles that encircle the LED, as shown by way of example in **FIG. 7.** Additional bristle tufts or an inner ring of bristle tufts might, however, be provided.

Referring again to **FIG. 5,** a switch **50** is provided to control operation of the illuminated electric toothbrush and is operatively connected to the motor **32.** The switch **50** is also configured to operate the one or more LEDs of the toothbrush. Such operation is preferably momentary or continuous. When the switch **50** is closed, a circuit comprising wire **54** is completed between a standard battery **60** provided within the hollow portion **30** of the handle **12** and the motor and LED **75.**

In embodiment of the toothbrush **400** the LED **75** can be placed on the head **416** so that it is between static bristle holder(s) **422** and movable bristle holder **420** and not aligned with an axis of rotation/oscillation of a moving bristle holder, as shown by way of example in **FIG. 8** wherein the bristles have been deleted for clarity. Head **416** is connected to handle (not shown) by neck **414.**

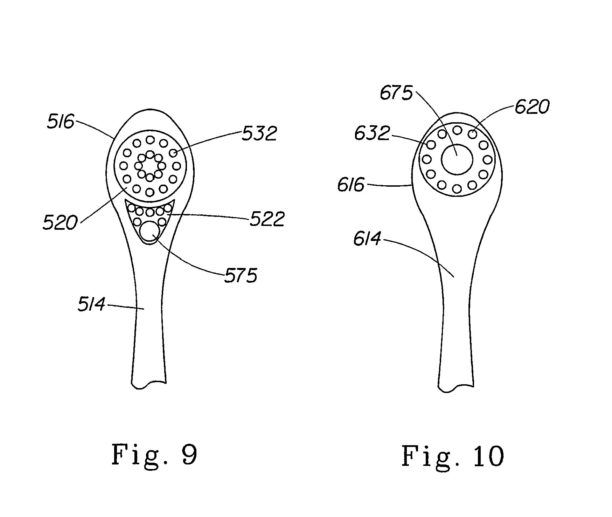

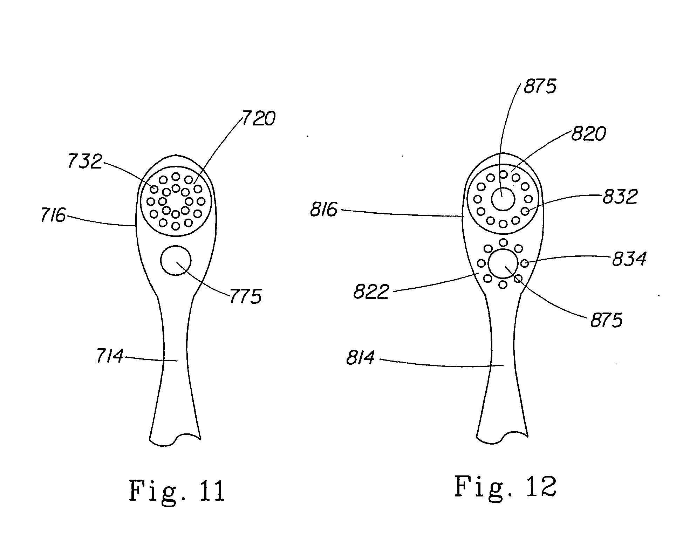

**FIGS. 9-12** illustrate other head, bristle holder and bristle configurations for illuminated electric toothbrushes, all of which contain one or more LEDs. **FIG. 9** illustrates a head **516** and a neck **514.** It will be appreciated that the neck **514** extends between the head **516** and a handle of the toothbrush (not shown). Disposed on the head **516** is a single moving bristle holder **520** having a plurality of bristles tufts **532** disposed thereon. Disposed on a second bristle holder **522** is a LED **575. FIG. 10** depicts another head **616** in accordance with the present invention having a plurality of bristles **632** disposed thereon. The head **616** comprises a single bristle holder **620** having LED **675** disposed therein. Neck **614** extends between head **616** and handle (not shown). **FIG. 11** depicts yet another head **716** having a single bristle holder **720** having bristles **732** disposed thereon. A LED **775** is disposed adjacent the bristle holder **720** on the head **716.** The LED **775,** however, is not disposed on bristle holder. **FIG. 12** depicts still another head **816** having a first bristle holder **820** that moves and a second bristle holder **822** that is fixed or stationary. Both bristle holders have LEDs **875** disposed thereon. The first bristle holder **820** has a plurality of bristle tufts **832** that encircle the LED **875** disposed thereon, and the second bristle holder **822** has a plurality of bristle tufts **834** that encircle the LED **875** disposed thereon. Neck **814** extends between head **816** and a handle (not shown).

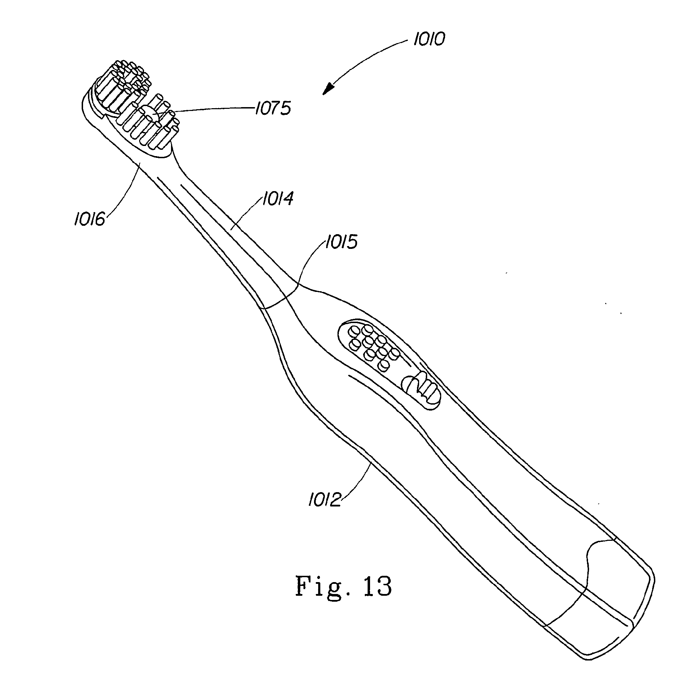

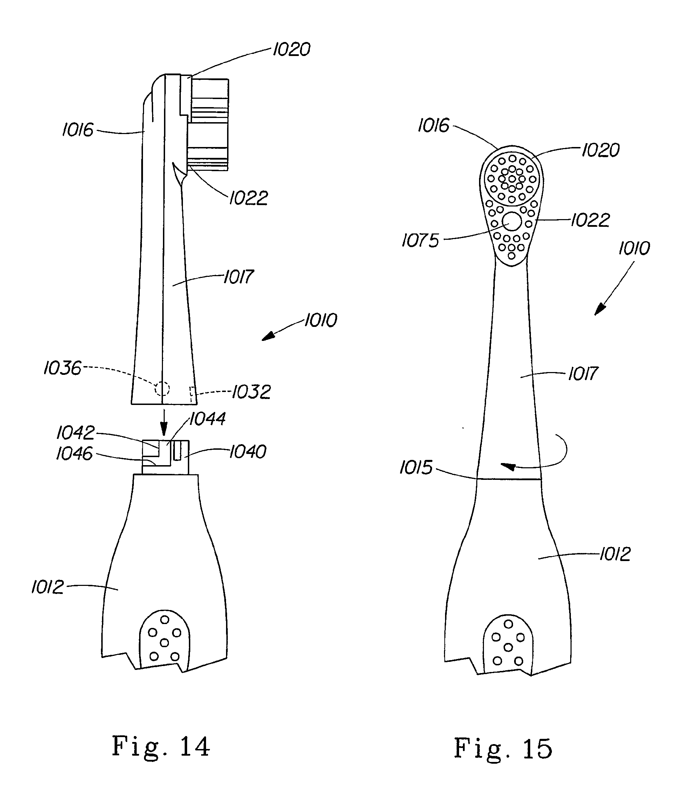

As shown in **FIG. 13,** an embodiment of the illuminated electric toothbrush **1010** having a head **1016,** neck **1014,** and a handle **1012.** Disposed on the head **1016** is LED **1075.** The neck and handle are releasably connected at **1015** and contain corresponding structures for their physical engagement and for establishing electrical communication between the LED and the power source. Referring now to **FIGS 14** and **15,** the head **1016** further includes a moving bristle holder **1020** and a static bristle holder **1022.** Disposed on the static bristle holder **1022** is a LED **1075.**

The neck 1017 separates from the handle 1012 at joint 1015. The neck 1017 has two small pins or projections 1036 [in phantom] located inside the neck end portion 1032. The small projections are dimensioned to fit into L-shaped slots **1042** found on a mating end portion **1040** of the handle **1012.** The width of the L-shaped slots **1042** is slightly wider than the width of the small projections to enable the L-shaped slots to receive the small projections. The depth of the L-shaped slots is substantially equal to the height of the small projections so that the L-shaped slots can receive the small projections.

To connect the head and neck to the handle, the user aligns the small projections with a top surface **1044** of the L-shaped slots. The user pushes or presses the head **1016** down so that the small projections contact a bottom surface **1046** of the L-shaped slots **1042.** When the small projections have contacted the bottom surface **1046** of the L-shaped slots, the user then turns the head **1016** and/or the neck **1017** approximately 90 degrees with respect to the handle **1012** locking the head into place, as seen in **FIGS. 14** and **15.** A top surface of each of the projections becomes locked under a top surface of each of the L-shaped slots **1042.** The user thus exerts a press-and-twist action on the cooperating pins and guide slots to put the head into a fully attached disposition on the handle and realize a locking engagement between the two.

**One** or more electrical contacts are provided along the mating region of the neck and the handle to provide a releasable electrical connection there between.

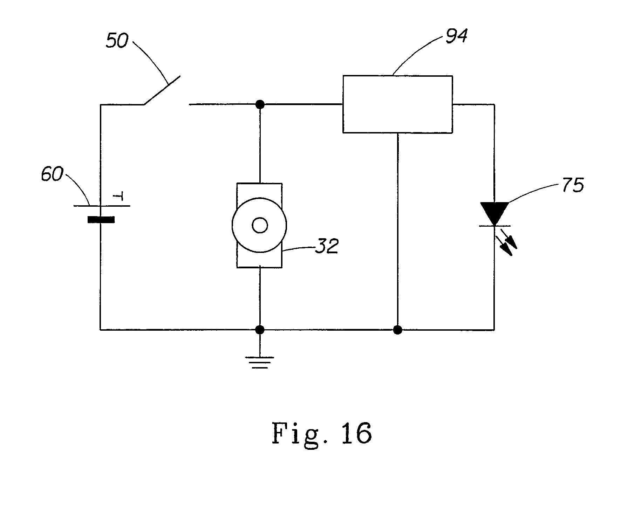

**FIG. 16** illustrates a schematic of an electrical configuration for the present invention. In this configuration, the LED **75** and the motor **32** are powered or activated concurrently with one another by switch **50** and power source **60.** Due to the fact that an LED is included and the power provided by the battery may exceed that which is desired for the LED, it may be desirable to include a standard voltage or current driver **94** which can provide a constant voltage or current output to the LED despite changes to the input voltage or current, especially as the voltage or current output from a battery tends to decrease over time. While the schematic shown in **FIG. 16** is one embodiment, other configurations can be provided. For example, separate switches might be provided to separately activate the LED and the motor. More than one LED might be provided. LEDs having different spectral, photometric, radiometric, and colorimetric characteristics (e.g., different dominant wavelengths, peak wavelengths, radiometric power, etc.) might be provided to accommodate multiple uses in a single electric toothbrush. This can also be accomplished using an LED having multiple dices (as shown in **FIG. 2- 2b).**

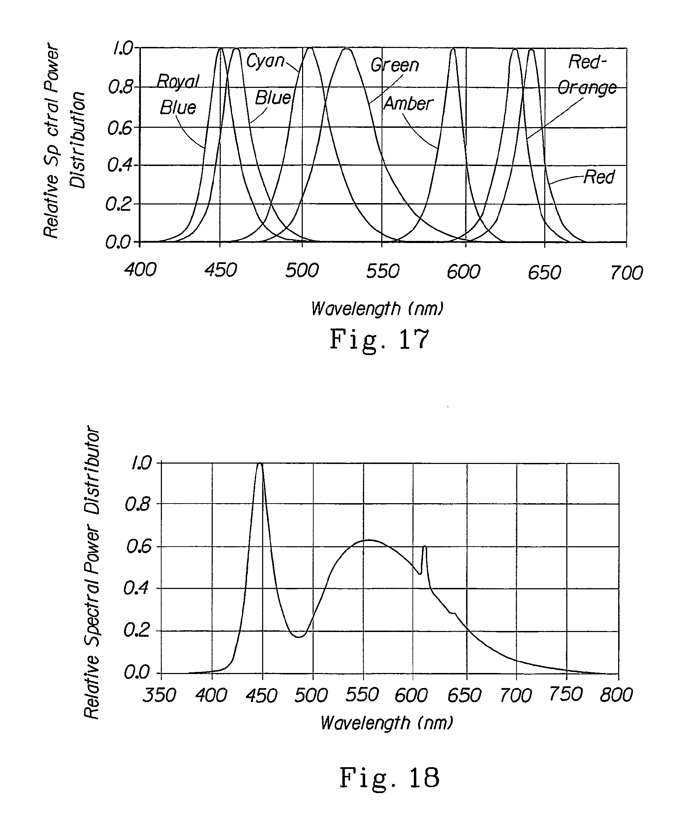

**FIGS. 17** and **18** illustrate spectral distributions for various colors of commercially available LED light emitting unit used in the electric toothbrushes described herein. These spectral distribution graphs are for Luxeon™ 1- watt emitter LEDs, however these distribution patterns may be achieved with other light emitting units. Specifically, **FIG. 17** is a graph of the relative spectral power distribution for various colors of LEDs. **FIG. 17** illustrates the colors of royal blue, blue, cyan, green, amber, red-orange, and red. **FIG. 18** is the relative spectral power distribution for a white color LED.

For tooth bleaching as well as other applications, it is often desirable to utilize a LED that provides a generally or substantially uniform distribution of radiometric power so that each tooth receives about the same of amount of radiometric power over the tooth surface. Therefore, embodiments of the inventive toothbrush comprise light radiation patterns having lamberertian or bell-shaped patterns, such as shown by way of example in **FIG. 19.** Other radiation patterns, such as the bat-wing pattern may also be utilized. As discussed above, however, the LED may provide a wide variety of light radiation patterns in accordance with the present invention.

The bristles of the bristle holders can be arranged to minimally interfere with the light emitted from the LED. Bristles can have a height of at least about 0.5, 0.6, 0.7, 0.8, 0.9 and/or 1.0 cm, and/or less than about 2.0, 1.5, 1.4, 1.3, 1.2, 1.1, and/or 1.0 cm. However, it is contemplated that the toothbrushes of the present invention may utilize bristle arrangements or materials that interact with the light emitted from the LED. For example, bristles and/or the top surface of the bristle holder located immediately adjacent the LED could include a reflective coating, such as nickel or chrome, to assist with directing light away from the head and toward the tooth surfaces. Alternately, bristles near the LED could be formed from a transparent or translucent material to further promote the transmission of light to the brushing area. The bristles might also be colored, pigmented, or dyed to generally match the color of the light emitted by the LED. In this way, the bristle would not absorb, but reflect, the light emitted by the LED. In addition, the use of a reflective shield that assists with directing light toward the tooth or gum surfaces which is placed around or near the LED might be utilized.

As previously noted herein, the embodiment toothbrushes with LED may be used in conjunction with a whitening composition for whitening teeth, and in particular, for enhancing or accelerating the whitening function of the composition by irradiating the brushing region either prior to, during, or after application of the whitening composition. A kit can be provided comprising the illuminated electric toothbrush, and a composition comprising peroxide.

Color in organic compounds is usually attributed to chromophores, which are unsaturated groups that can undergo π electronic transitions. Light can activate stain chromophores (undergo electronic transition), and reduce activation energy barrier making them more susceptible to attack by bleaching. In other words, activation of color bodies via light may enhance peroxide bleaching. Similarly, stain chromophores become more susceptible to abrasive whitening because of light treatment which results in faster and better whitening. Bleaching agents penetrate into the pores in enamel and dentin, and, therefore, both extrinsic and intrinsic color stains can be degraded and removed.

A wide variety of tooth whitening compositions may be used in combination with the electric toothbrushes described herein. The tooth whitening compositions may contain a bleaching agent, an abrasive agent, pH modifiers or any other agent that acts upon the chromophores of the teeth by mechanical or chemical action or a combination thereof. The tooth whitening composition can be provided in the form of a solution, paste, gel, viscous liquid, solid, or other suitable form. Illustrative bleaching agents include an oxygen radical or hydrogen radical-generating compound such as metal ion free peroxides, organic peroxides, and metal ion containing peroxides. Specific, non-limiting examples of bleaching agents include peroxides, metal chlorites, perborates, percarbonates, peroxyacids, persulfates, compounds that form the preceding compounds in situ, and combinations thereof. Suitable peroxide compounds include hydrogen peroxide, urea peroxide, calcium peroxide, carbamide peroxide, and mixtures thereof. In one embodiment the bleaching agent is carbamide peroxide. Suitable metal chlorites include calcium chlorite, barium chlorite, magnesium chlorite, lithium chlorite, sodium chlorite, potassium chlorite, and mixtures thereof. Additional bleaching agents also include hypochlorite and chlorine dioxide. In one embodiment the bleaching agent is selected from sodium chlorite, peroxide, sodium percarbonate, oxones, and mixtures thereof. The starting bleaching agent can be aqueous or solid material.

The amount of bleaching agent in the whitening or bleaching composition may vary. For example, the bleaching agent could be present in an amount of about 0.5 to about 60 weight percent, based on the total amount of the tooth whitening composition. If hydrogen peroxide is the bleaching agent, according to one particular embodiment, it may be present in about 0.5 to about 40 weight percent, especially about 7 to about 15 weight percent, based on the total amount of the tooth whitening composition. If carbamide peroxide is the bleaching agent, according to one particular embodiment, it may be present in about 10 to about 60 weight percent, based on the total amount of tooth whitening composition. Typically, the radiant energy from the LED is applied while the composition is in contact with the tooth, however, may be applied prior to or after application of the tooth whitening composition.

The illuminated electric toothbrush can be packaged as a kit one or more replaceable heads containing a LED. Although the handle is discussed as preferably battery powered, the invention also includes other well known power supplies such as corded for outlet connection or rechargeable batteries and an associated brush holder/charger (not shown).

As discussed above, the various embodiments of the illuminated electric toothbrush may be used in combination with a whitening composition. A representative method of whitening teeth is as follows. After obtaining the illuminated toothbrush and composition, the composition is applied to the dental surface, i.e. teeth, to be whitened. Preferably, such application is performed by depositing an effective amount of the composition on the bristle holder of the toothbrush, and then applying the composition to the desired surfaces to be whitened. Generally, this latter step is performed in like fashion as brushing one's teeth. Alternatively, the tooth whitening composition might be brushed, painted, or applied to the teeth with an applicator strip. The light emitting unit of the toothbrush is then activated and the light emitted there from is directed to the applied composition. It will be understood that the various whitening techniques of the present invention include variant strategies in which the light is directed to the dental surface before, during, and after application of the composition to the dental surface. Preferably, a brushing operation is then performed while the light continues to irradiate the composition applied to the dental surface of interest.

This whitening process is merely exemplary. The present invention includes a wide array of whitening techniques. Additionally, it is contemplated that a conventional brushing operation may be performed prior to, during, or subsequent to a whitening operation.

The present invention has been described with reference to multiple embodiments. Obviously, modifications and alterations will occur to others upon a reading and understanding of this specification. For example, any number of bristle holders and bristle patterns can be utilized with the present invention along with one more LED. It is intended to include all such modifications and alterations insofar as they come within the scope of the appended claims.

**Claims**

1. An illuminated electric toothbrush (10, 400, 1010) comprising:

   (a) a handle (12, 1012) a head (16, 316, 416, 516, 616, 716, 816, 1016) and a neck (14, 314, 414, 514, 814, 1014) extending between said handle and said head, said handle having a hollow interior region (30) said head having bristle (632, 732 832, 834) disposed thereon, and said electric toothbrush having a longitudinal axis (19)
   (b) at least one light emitting diode (75, 575, 675, 775, 875, 1075) disposed on said head, wherein said light emitting diode is powered by an electric current; and
   (c) one or more bristle holders (20, 320, 420, 520, 820, 1020) disposed on said head, said bristle holders having a plurality of bristles disposed thereon and defining a main bristle direction, wherein
   said bristle holder (20, 320, 420, 520, 820, 1020) is disposed movable on said head and operatively connected to a motor disposed in the hollow interior region (30) of the handle (12, 1012) by a drive shaft (44),
   **characterized in that** said at least one light emitting diode (75, 575, 675, 775, 875, 1075) has a flux density between 30 mW/cm$^2$ to 300 mW/cm$^2$ at a detector distance (1200) of 0.68 cm and at a detector aperture (1201) area of 0.46 cm$^2$, wherein said light emitting diode has an emission temperature of less than 43°C at an emission distance (1303) of 0.68 cm and at an emission time of 2 minutes, and wherein said at least one light emitting diode emits light having a half angle of less than 50° with the principle light emission direction being parallel to the main bristle direction.

2. The illuminated electric toothbrush according to the preceding claim, wherein said toothbrush further comprises a current driver (94) which delivers a current greater than 35 mA to said light emitting diode, and wherein said motor said driver and said battery and said light emitting diode are part of an electrical circuit.

3. The illuminated electric toothbrush of Claim 1, wherein said current is a continuous forward current greater than 35 mA.

4. The illuminated electric toothbrush of Claim 1, wherein said current is a pulsed forward current having a peak greater than 100 mA.

5. The illuminated electric toothbrush according to any of the preceding claims, wherein said light emitting diode is substantially surrounded by one ring of bristles.

6. The illuminated electric toothbrush according to any of the preceding claims, wherein said light emitting diode comprises at least two semi-conductor substrates (5, 17) having light emitting proprerties, and one common lens (3).

7. The illuminated electric toothbrush according to any of the preceding claims, wherein said light emitting diode emits light having a wavelength of from 440 nm to 480 nm.

**Patentansprüche**

1. Beleuchtete elektrische Zahnbürste (10, 400, 1010), umfassend:

   (a) einen Griff (12, 1012), ein Kopfstück (16, 316, 416, 516, 616, 716, 816, 1016) und einen Hals (14, 314, 414, 514, 814, 1014), der sich zwischen dem Griff und dem Kopfstück erstreckt, wobei der Griff einen hohlen Innenbereich (30) aufweist, wobei das Kopfstück darauf angeordnete Borsten (632, 732, 82, 834) aufweist und die elektrische Zahnbürste eine Längsachse (19) aufweist

(b) mindestens eine Leuchtdiode (75, 575, 675, 775, 875, 1075), die auf dem Kopfstück angeordnet ist, wobei die Leuchtdiode durch einen elektrischen Strom betrieben ist, und

(c) einen oder mehrere Borstenhalter (20, 320, 420, 520, 820, 1020), die auf dem Kopfstück angeordnet sind, wobei die Borstenhalter mehrere darauf angeordnete Borsten aufweisen und eine Borstenhauptrichtung definieren, wobei

der Borstenhalter (20, 320, 420, 520, 820, 1020) auf dem Kopfstück beweglich angeordnet ist und mit einem Motor, der in dem hohlen Innenbereich (30) des Griffs (12, 1012) angeordnet ist, durch eine Antriebswelle (44) funktionsmäßig verbunden ist,

**dadurch gekennzeichnet, dass**

die mindestens eine Leuchtdiode (75, 575, 675, 775, 875, 1075) eine Flussdichte zwischen 30 mW/cm$^2$ bis 300 mW/cm$^2$ bei einem Detektorabstand (1200) von 0,68 cm und bei einer Detektoröffnungs- (1201) Fläche von 0,46 cm$^2$ aufweist, wobei die Leuchtdiode eine Emissionstemperatur von weniger als 43 °C bei einem Emissionsabstand (1303) von 0,68 cm und bei einer Emissionszeit von 2 Minuten aufweist und wobei die mindestens eine Leuchtdiode Licht mit einem Halbwinkel von weniger als 50° emittiert, wobei die Lichtemissions-Hauptrichtung zu der Borstenhauptrichtung parallel ist.

2. Beleuchtete elektrische Zahnbürste nach dem vorstehenden Anspruch, wobei die Zahnbürste ferner eine Stromsteuerung (94) umfasst, die einen Strom von mehr als 35mA an die Leuchtdiode liefert, und wobei der Motor, die Steuerung und die Batterie und die Leuchtdiode Teil eines elektrischen Schaltkreises sind.

3. Beleuchtete elektrische Zahnbürste nach Anspruch 1, wobei der Strom ein Gleichstrom in Durchlassrichtung von mehr als 35 mA ist.

4. Beleuchtete elektrische Zahnbürste nach Anspruch 1, wobei der Strom ein Impulsstrom in Durchlassrichtung mit einer Spitze von mehr als 100 mA ist.

5. Beleuchtete elektrische Zahnbürste nach einem der vorstehenden Ansprüche, wobei die Leuchtdiode von einem Ring von Borsten im Wesentlichen umgeben ist.

6. Beleuchtete elektrische Zahnbürste nach einem der vorstehenden Ansprüche, wobei die Leuchtdiode mindestens zwei Halbleitersubstrate (5, 17) mit Lichtemissionseigenschaften und eine gemeinsame Linse (3) umfasst.

7. Beleuchtete elektrische Zahnbürste nach einem der vorstehenden Ansprüche, wobei die Leuchtdiode Licht mit einer Wellenlänge von 440 nm bis 480 nm emittiert.

**Revendications**

1. Brosse à dents électrique éclairée (10, 400, 1010) comprenant :

(a) un manche (12, 1012), une tête (16, 316, 416, 516, 616, 716, 816, 1016), et un col (14, 314, 414, 514, 814, 1014) s'étendant entre ledit manche et ladite tête, ledit manche ayant une région intérieure creuse (30) ladite tête ayant des poils (632, 732, 832, 834) disposés dessus, et ladite brosse à dents électrique ayant un axe longitudinal (19)

(b) au moins une diode électroluminescente (75, 575, 675, 775, 875, 1075) disposée sur ladite tête, dans laquelle ladite diode électroluminescente est alimentée par un courant électrique ; et

(c) un ou plusieurs supports de poils (20, 320, 420, 520, 820, 1020) disposés sur ladite tête, lesdits supports de poils ayant une pluralité de poils disposés dessus et définissant une direction principale de poils, dans laquelle

ledit support de poils (20, 320, 420, 520, 820, 1020) est disposé mobile sur ladite tête et fonctionnellement attaché à un moteur disposé dans la région intérieure creuse (30) du manche (12, 1012) par un arbre d'entraînement (44),

**caractérisée en ce que**

ladite au moins une diode électroluminescente (75, 575, 675, 775, 875, 1075) a une densité de flux comprise entre 30 mW/cm$^2$ et 300 mW/cm$^2$ à une distance de détecteur (1200) de 0,68 cm et à une aire d'ouverture de détecteur (1201) de 0,46 cm$^2$, dans laquelle ladite diode électroluminescente a une température d'émission inférieure à 43 °C à une distance d'émission (1303) de 0,68 cm et à un temps d'émission de 2 minutes, et dans laquelle ladite au moins une diode électroluminescente émet de la lumière ayant un demi-angle inférieur à 50° avec la direction principale d'émission de lumière étant parallèle à la direction principale des poils.

2. Brosse à dents électrique éclairée selon la revendication précédente, où ladite brosse à dents comprend en outre un excitateur de courant (94) qui délivre un courant supérieur à 35 mA à ladite diode électroluminescente, et dans laquelle ledit moteur, ledit excitateur et ladite batterie et ladite diode électroluminescente font partie d'un circuit électrique.

3. Brosse à dents électrique éclairée selon la revendication 1, dans laquelle ledit courant est un courant direct continu supérieur à 35 mA.

4. Brosse à dents électrique éclairée selon la revendication 1, dans laquelle ledit courant est un courant direct pulsé ayant un pic supérieur à 100 mA.

5. Brosse à dents électrique éclairée selon l'une quelconque des revendications précédentes, dans laquelle ladite diode électroluminescente est essentiellement entourée par un anneau de poils.

6. Brosse à dents électrique éclairée selon l'une quelconque des revendications précédentes, dans laquelle ladite diode électroluminescente comprend au moins deux substrats semi-conducteurs (5, 17) ayant des propriétés d'émission de lumière, et une lentille commune (3).

7. Brosse à dents électrique éclairée selon l'une quelconque des revendications précédentes, dans laquelle ladite diode électroluminescente émet une lumière ayant une longueur d'onde allant de 440 nm à 480 nm.

Fig. 1

Fig. 2

Fig. 2A

Fig. 2B

Fig. 2C

Fig. 3

Fig. 4

Fig. 5

EP 1 663 058 B1

Fig. 6

EP 1 663 058 B1

Fig. 6A

Fig. 7

Fig. 8

Fig. 9

Fig. 10

Fig. 11

Fig. 12

Fig. 13

Fig. 14

Fig. 15

Fig. 16

Fig. 17

Fig. 18

Fig. 19

Fig. 20

Fig. 21

EP 1 663 058 B1

Fig. 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9510243 A
- US 20030059738 A
- US 20030126699 A
- US 20030084525 A
- US 20030084524 A
- US 20030084526 A
- WO 03063723 A
- WO 03063722 A
- US PNS6360395 A
- US 5617601 A